(19) Europäisches Patentamt
European Patent Office
Office européen des brevets

(11) Veröffentlichungsnummer: **0 012 224 B1**

# (12) EUROPÄISCHE PATENTSCHRIFT

(45) Veröffentlichungstag der Patentschrift: **18.01.89**

(21) Anmeldenummer: **79104424.1**

(22) Anmeldetag: **09.11.79**

(51) Int. Cl.⁴: **C 07 C 33/02**, C 07 C 47/46, C 07 C 29/00, C 07 C 45/69, C 07 C 29/88, C 07 C 91/26, C 07 C 87/24, C 07 C 85/18, C 07 C 89/00

(54) **Verfahren zur Herstellung von Riechstoffgemischen.**

(30) Priorität: **13.11.78 US 960152**

(43) Veröffentlichungstag der Anmeldung: **25.06.80 Patentblatt 80/13**

(45) Bekanntmachung des Hinweises auf die Patenterteilung: **18.01.89 Patentblatt 89/3**

(84) Benannte Vertragsstaaten: **CH DE FR GB NL**

(56) Entgegenhaltungen:
**DE-A-2 403 631**
**FR-A-1 456 900**
**FR-A-2 351 075**
**GB-A-868 850**
**US-A-3 060 237**
**US-A-3 932 539**
**US-A-4 007 137**

**S. ARCTANDER: "Perfume and flavor chemicals", 1969, Montclair, N.J., US**

(73) Patentinhaber: **L. Givaudan & Cie Société Anonyme, Patentdienst Postfach, CH-4002 Basel (CH)**

(72) Erfinder: **Chalk, Alan J., 5 Duchess Drive Fayson Lakes, Kinnelon, N.J., 07405 (US)**
Erfinder: **Magennis, Steven A., 12 New Street, Wayne, N.J. 07470 (US)**

(74) Vertreter: **Lederer, Franz, Dr. et al, Van der Werth, Lederer & Riederer Patentanwälte Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft ein neues und verbessertes Verfahren zur Herstellung des Gemisches von 3- und 4-(4-Methyl-4-hydroxyamyl)$\Delta^3$-cyclohexencarboxaldehyd aus dem leicht zugänglichen Myrcen. Bei diesem neuen Verfahren fällt ein neues Myrcenol-Ocimenol-Zwischenprodukt an, welches im wesentlichen frei von trans-Ocimenol ist.

Es ist bekannt, dass das Diels-Alder-Addukt von Myrcenol und Acrolein aus einem Gemisch von I und II gemäss:

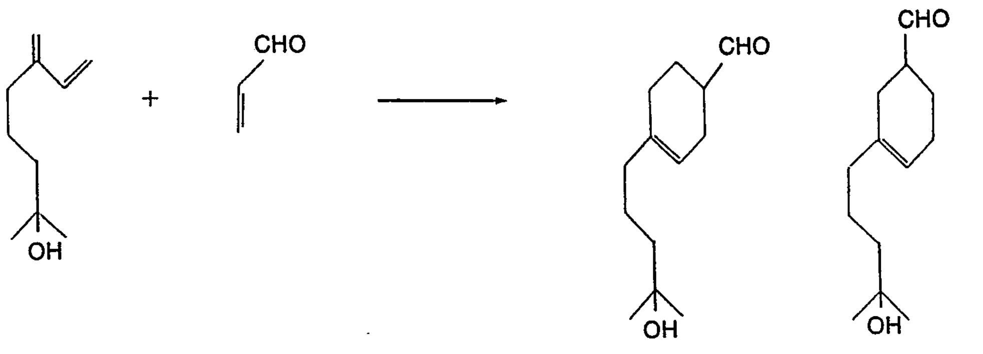

Myrcenol Acrolein Hauptkomponente I Nebenprodukt II

besteht. Wenn die Reaktion ohne Anwesenheit eines Katalysators (thermisch) durchgeführt wird, beträgt das Verhältnis von I:II ungefähr 3:1. Wird die Reaktion aber in Anwesenheit eines Katalysators durchgeführt, überwiegt I noch mehr, das Verhältnis ist sogar eher 9:1. Der Stand der Technik bezüglich katalysierter und nicht katalysierter Reaktion wird in der U.S. Patentschrift 4 007 137 abgehandelt.

Bekannt sind auch Verfahren, um Myrcen in Myrcenol überzuführen. Um diesen Prozess zu bewerkstelligen, muss der Dien-Teil im Myrcen vor der Hydratisierung der isolierten Doppelbindung geschützt werden. So wird beispielsweise in der U.S. Patentschrift 3 176 022 ein Prozess beschrieben, worin durch Reaktion von Myrcen mit Schwefeldioxid ein cyclisches Sulfon hergestellt wird, wobei der Dien-Teil mit dem Schwefeldioxid gemäss Diels-Alder reagiert. Das gebildete Myrcensulfon wird hierauf hydratisiert und der resultierende tertiäre Alkohol erhitzt, wobei das Dien regeneriert wird und das gewünschte Myrcenol entsteht.

Die U.S. Patentschrift 3 932 529 lehrt die Herstellung eines Gemisches von Terpenalkoholen, das im wesentlichen aus Myrcenol und mindestens 20% cis-Ocimenol und trans-Ocimenol besteht. Gemäss dieser Literaturstelle wird Myrcen zu einem Gemisch von Linalylchlorid (ca. 10%), Neryl- und Geranylchlorid hydrochloriniert. Diese Chloride werden mit einem tertiären Amin (Trimethylamin) umgesetzt, wobei ein quaternäres Ammoniumsalz anfällt, das dann zu einem Gemisch der

cis- und trans-3,7-Dimethyl-7-hydroxy-2-octen-1-yl-trimethylammoniumchloride

hydratisiert wird. Die Chloride werden hierauf in die Hydroxide übergeführt und thermisch zersetzt (Hofmann'scher Abbau), wobei ein Gemisch von Myrcenol, cis-Ocimenol und trans-Ocimenol anfällt.

Währenddem über das Sulfon-Zwischenprodukt (U.S.P. 3 176 022) reines Myrcenol anfällt, wird über das quaternäre Ammoniumsalz (U.S.P. 3 932 539) ein Gemisch von Myrcenol und Ocimenol erhalten. Ein solches Gemisch von Myrcenol, cis-Ocimenol und trans-Ocimenol ist für die Herstellung der Diels-Alder-Addukte, der 3- und 4-(4-Methyl-4-hydroxyamil)-$\Delta^3$-cyclohexancarboxaldehyde ungeeignet, da es bekannt ist (U.S.P. 3 758 590), dass Ocimenol mit Acrolein unter Bildung eines Addukts reagiert. Es entsteht auf diese Weise ein Addukt.

Es muss in diesem Zusammenhang festgehalten werden, dass, obschon U.S.P. 3 758 590 keine Angaben liefert, ob ausgehend von cis-Ocimenol und/oder trans-Ocimenol Addukte entstehen, es wichtig ist zu wissen, dass nur trans-Ocimenol in der Lage ist, mit Acrolein ein Addukt zu bilden.

Die vorliegende Erfindung betrifft nun ein neues und verbessertes Verfahren zur Herstellung von Diels-Alder-Addukten, nämlich von

3- und 4-(4-Methyl-4-hydroxyamyl)-$\Delta^3$-cyclohexancarboxaldehyd

ausgehend von Myrcenol, welches aus Myrcen zugänglich ist. Die neue Route kann wie folgt illustriert werden:

Schema I

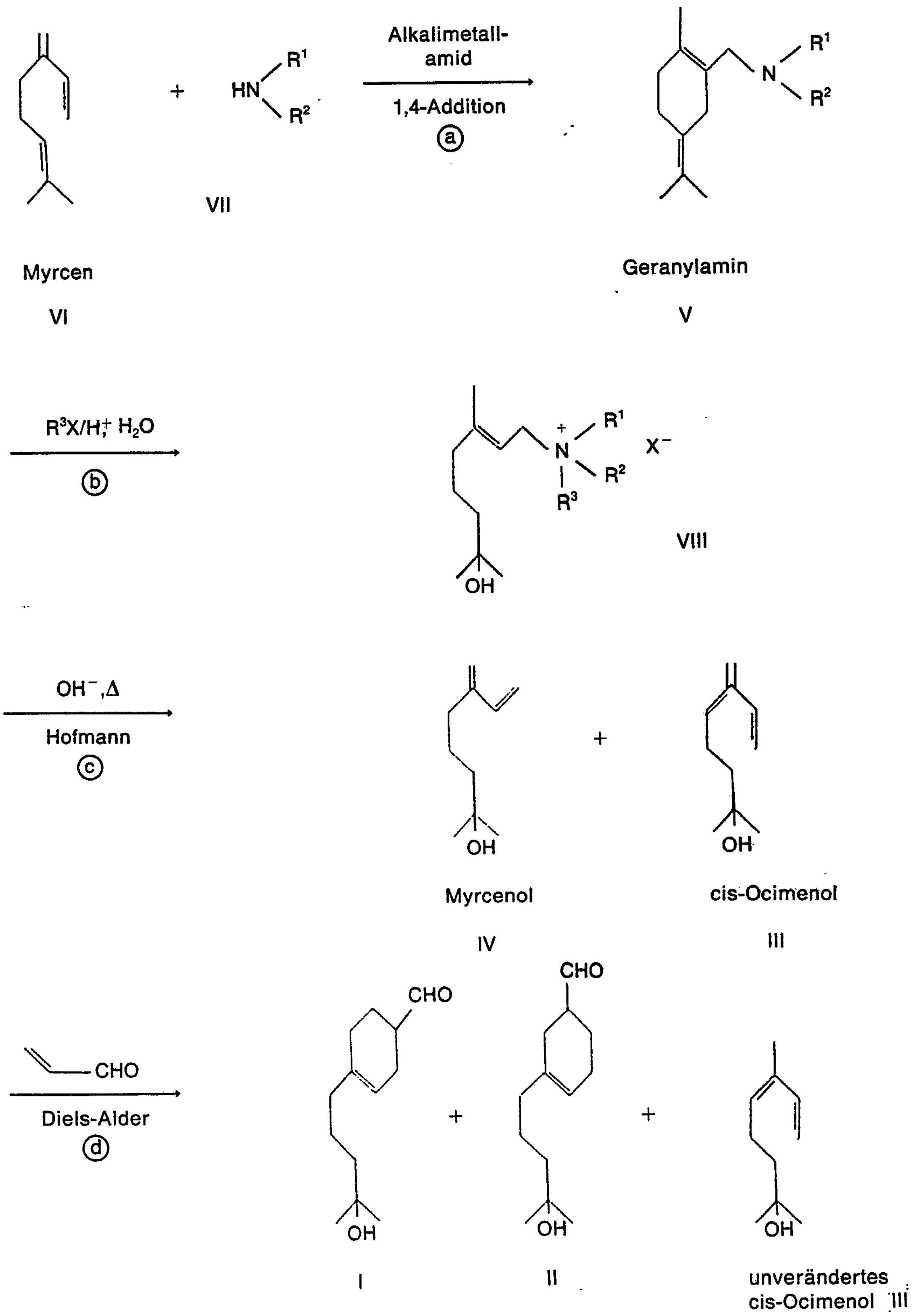


Myrcen VI

VII

Geranylamin V

VIII

Myrcenol IV

cis-Ocimenol III

I

II

unverändertes cis-Ocimenol III

Im obigen Schema I handelt es sich beim Amin um ein sekundäres Amin, worin $R^1$ und $R^2$ Äthyl oder einen höheren normalen Alkylrest (beispielsweise bis zu 8 C enthaltend) darstellen oder zusammen einen cycloaliphatischen Rest bilden, der einen $C_{1-8}$-Alkyl-Substituenten an einem der Kohlenstoffatome, die an das Stickstoffatom gebunden sind, trägt. $R^3$ stellt eine Alkylgruppe dar, zweckmässigerweise Methyl bis Octyl, Benzyl (unsubstituiert oder substituiert), und ähnliches. X kann Chlor, Brom, Jod, Methylsulfat, etc. darstellen. [Das Anion $X^-$ kann auch für $OH^-$ stehen]. Die an die verschiedenen Substituenten gestellten Anforderungen werden weiter unten erläutert.

Der Erfolg des oben erläuterten Verfahrens hängt von einer Anzahl überraschender und unerwarteter Tatsachen ab. Der Einfluss dieser Tatsachen auf den Erfolg des erfindungsgemässen Verfahrens ist aus den unten stehenden Erläuterungen ersichtlich.

Aus der Stufe d des Schemas I ist ersichtlich, dass, wenn ein Gemisch von Myrcenol und cis-Ocimenol mit Acrolein unter Diels-Alder-Bedingungen umgesetzt wird, ausgehend von cis-Ocimenol kein Addukt entsteht. Es folgt deshalb, dass ein Gemisch von Myrcenol und cis-Ocimenol ein geeignetes Ausgangsmaterial für eine wirtschaftliche Herstellung des Gemisches von I und II darstellt, da das nicht reagierende cis-Ocimenol aus dem Reaktionsgemisch leicht durch Destillation abgetrennt werden kann.

Die kritische Stufe im erfindungsgemässen Verfahren liegt im Schritt c. Es ist überraschend und unerwartet, dass der Hofmann'sche Abbau eines

E-3,7-Dimethyl-7-hydroxy-2-octen-1-yl-trialkylammonium-hydroxids (auch 7-Hydroxygeranylamin-quaternäres-hydroxid genannt)

in dem Sinne abläuft, dass ein Gemisch von Myrcenol und cis-Ocimenol entsteht, welches im wesentlichen frei von trans-Ocimenol ist. Der Begriff «im wesentlichen frei von» wird im vorliegenden Fall benützt, um anzuzeigen, dass weniger wie 3% des Materials im Produkt auftreten.

Der Begriff «Hofmann'scher Abbau» wie er hier benützt wird, bezieht sich auf die thermische Zersetzung eines quaternären Ammoniumhydroxids, wobei ein Olefin entsteht. Ein geeigneter diesbezüglicher Übersichtsartikel ist derjenige von A.C. Cope und E.R. Trumbull, Organic Reactions, Band 11, Seite 317, John Wiley and Sons, New York, 1960.

Wenn andererseits ein

Z-3,7-Dimethyl-7-hydroxy-2-octen-1-yl-trialkylammoniumhydroxid (auch als 7-Hydroxynerylamin-quaternäres-hydroxid bezeichnet)

den Bedingungen eines Hofmann'schen Abbaus unterworfen wird, entsteht ein Produkt, das aus einem Gemisch von trans-Ocimenol und Myrcenol besteht, worin das trans-Ocimenol die Hauptkomponente darstellt. Dieses Gemisch ist im wesentlichen frei von cis-Ocimenol.

Es folgt daraus, dass, wenn das

3,7-Dimethyl-7-hydroxy-2-octen-1-yl-trialkylammoniumhydroxid

einen wesentlichen Anteil an Z-Isomerem enthält, der Hofmann'sche Abbau ein trans-Ocimenol liefert, in dem dieses trans-Ocimenol ungefähr zwei Drittel des cis-Isomeren beträgt. Jede nun folgende Diels-Alder-Reaktion mit Acrolein würde also ein Produkt liefern, das mit trans-Ocimenol/Acrolein-Addukt verunreinigt ist. Es ist demgemäss wünschenswert, den Anteil an Z-Isomeren im

3,7-Dimethyl-7-hydroxy-2-octen-2-yl-trialkylammoniumhydroxid

zu minimalisieren, um ein Myrcenol/Acrolein-Addukt zu erhalten, das im wesentlichen frei von trans-Ocimenol/Acrolein-Addukt ist.

Eine andere überraschende und unerwartete Tatsache besteht darin, dass die 1,4-Addition von gewissen sekundären Aminen an Myrcen, wie gemäss Schritt a des Schemas I illustriert, kontrolliert werden kann, sodass ein Produkt anfällt, das im wesentlichen aus dem entsprechenden Geranylamin (dem E-Isomeren) mit nur kleinen Beimengungen an entsprechendem Nerylamin (dem Z-Isomeren) besteht. Im folgenden wird das E-Isomere (Struktur 2 des unten stehenden Schemas II) als Geranylamin und das Z-Isomere als Nerylamin (Struktur 3 im untenstehenden Schema II) bezeichnet.

Aufgrund der im Rahmen der vorliegenden Erfindung aufgefundenen Tatsachen werden dem Parfumeur gleichzeitig neue Riechstoffkompositionen zur Verfügung gestellt, Riechstoffkompositionen, die im bis anhin nicht oder in nicht genügender Menge zur Verfügung standen. Diese zusätzlichen Aspekte betreffen:

i) neue Myrcenol/cis-Ocimenol-Gemische, die im wesentlichen frei von trans-Ocimenol sind;

ii) ein Verfahren zur Herstellung von reinem cis-Ocimenol, welches gemäss Stand der Technik parfümistisch wertvoller als das trans-Isomere ist.

Arctander, Perfume and Flavor Chemicals (1969) beschreibt Ocimenol (No. 2389) und Myrcenol (No. 2284) als äusserst brauchbare Parfümeriematerialien, die beispielsweise Zitrusgerüche aufweisen. Die im Rahmen des vorliegenden Verfahrens anfallenden Gemische sind neu und ebenfalls nützlich in Parfums bzw. anderen Riechstoffkompositionen.

Das untenstehende Schema II illustriert die möglichen Isomeren, die aufgrund einer 1,4-Addition eines sekundären Amins an Myrcen entstehen können. Wie oben ausgeführt, besteht eines der Ziele der vorliegenden Erfindung darin, den Gang der Addition so zu führen, dass das entstehende Material hauptsächlich aus Geranylamin (2) besteht und nur kleine Beimengungen an Neranylamin (3) bzw. an iso-Isomerem (4) als Verunreinigungen anfallen.

Schema II

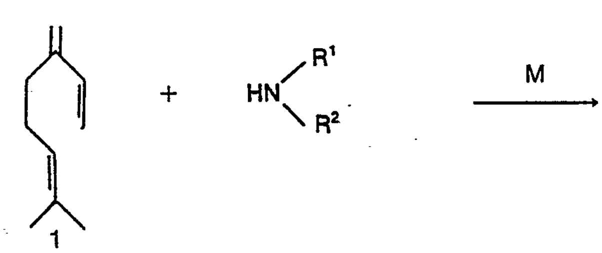


1

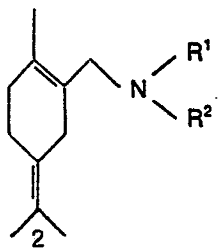
+
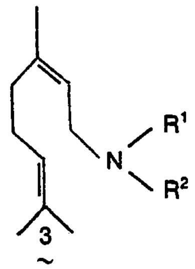
+
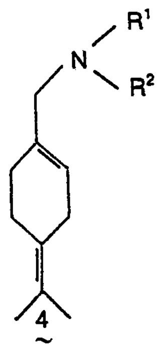

2 3 4

(V) (Vb)

Die Tabelle I liefert eine Anzahl Beispiele für die Reaktion gemäss Schema II. Diese Tabelle I zeigt klar, dass die Selektivität der 1,4-Addition eines sekundären Amins an Myrcen von der Natur des benützten Amins und in geringerem Ausmass auch vom Alkalimetall abhängt.

Tabelle I: Reaktion von Myrcen mit sekundären Aminen

| Beispiel | Amin | Myrcen/ Amin | Kataly- sator | Amin/ Kataly- sator | Tempe- ratur | Zeit Stunden | Produkte 2 | 3 | 4 | Aus- beute® % |
|---|---|---|---|---|---|---|---|---|---|---|
| | | Mole/Mol | | Mole/Mol | | | | | | |
| 1 | $Ät_2NH$ | 1,2 | BuLi | 28 | 25 | 72 | 96 | 2 | 2 | 87 |
| 2 | $Bu_2NH$ | 1,25 | Li | 12 | 60 | 24 | 97 | 1 | 2 | 75 |
| 3 | $Me_2NH$ | 1,5 | BuLi | 50 | 25 | 16 | 72 | 19 | 9 | 91 |
| 4 | $Me_2NH$ | 1,1 | Na | 57 | 40–50 | 24 | 63 | 20 | 17 | 71 |
| 5 | $Ät_2NH$ | 1,2 | Na | 59 | 50 | 4 | 90 | 5 | 5 | 76 |
| 6 | $Ät_2NH$ | 1,6 | Na | 59 | 50 | 4 | 87 | 8 | 5 | 67 |
| 7 | $Ät_2NH$ | 2,0 | Na | 59 | 50 | 4 | 88 | 7 | 5 | 62 |
| 8 | $iPr_2NH$ | 1,5 | BuLi | 12 | 60 | 30 | 95 | 4 | 1 | 26 |
| 9 | $iPr_2NH$ | 1,5 | Na | 12 | 50 | 21 | 96 | 3 | 1 | 17 |
| 10 | Piperidin | 2,2 | BuLi | 20 | 60 | 18 | 69 | 21 | 10 | 64 |
| 11 | $Bu_2NH$ | 1,25 | Na | 59 | 50 | 7 | 88 | 5 | 7 | 66 |
| 12 | * | 1,2 | BuLi | 20 | 50 | 28 | 97 | 0 | 3 | 85 |
| 13 | ** | 1,2 | BuLi | 10 | 60 | 96 | 97 | 2 | 1 | 47 |

* 2-Methylpiperidin
** 2,6-Dimethylpiperidin
® basierend auf Amin

Die Analyse der Tabelle I zeigt, dass die Zugabe von Dimethylamin am wenigsten selektiv wirkt (Beispiele 3 und 4), indem hier nur ungefähr zwei Drittel des Additionsproduktes aus dem gewünschten Geranylamin bestehen. Nahezu 20% des Additionsproduktes bestehen aus unerwünschtem Nerylamin. Nach der darauffolgenden Transformation gemäss Schema I entstände hier erwartungsgemäss ein Myrcenol-Ocimenol-Gemisch, das mehr als 10% des unerwünschten trans-Ocimenols enthält.

Aus diesem Grund werden vorzugsweise Diäthylamin oder höhere sekundäre Dialkylamine – siehe die Beispiele 1, 2, 5–9 und 11 – eingesetzt, welche zu Produkten führen, in denen das Geranylamin ungefähr 90% und der Gehalt an Nerylamin weniger wie 10% beträgt. Während alle diese Dialkylamine den erwünschten Grad von Selektivität liefern, sind doch diejenigen Dialkylamine, worin die Alkylgruppe eine normale Alkylgruppe (Äthyl, n-Propyl, n-Butyl, etc.) darstellt, bevorzugt gegenüber jenen, worin die Alkylgruppe verzweigt ist, also z.B. Isopropyl etc. darstellt, indem im ersteren Falle höhere Ausbeuten entstehen.

Aus der Tabelle I ist auch ersichtlich, dass, wenn cyclische sekundäre Amine eingesetzt werden, diese einen α-Substituenten zum Stickstoffatom tragen müssen. Wenn Piperidin verwendet wird, wie im Beispiel 10, ist die Selektivität ungefähr dieselbe wie im Falle von Dimethylamin. Ganz im Gegensatz dazu liefern 2-Methylpiperidin (Beispiel 12) und 2,6-Dimethylpiperidin (Beispiel (13) einen hohen Grad an Selektivität, eine Ausbeute, die vergleichbar derjenigen der Beispiele ist, in denen Diäthylamin eingesetzt wird.

Es wurde ferner gefunden, dass die Addition etwas selektiver ist, wenn das Alkalimetall Lithium und nicht Natrium ist. So geht aus der Tabelle I hervor, dass in denjenigen Beispielen, wo Diäthylamin verwendet wurde, und zusätzlich Lithium eingesetzt wurde (Beispiele 1 und 2), ungefähr 95% Geranylamin entstanden mit Beimengungen von weniger als 5% Nerylamin, während andererseits 90% Geranylamin entstanden und weniger als 10% Nerylamin, wenn Natrium zum Einsatz gelangte (Beispiele 5, 6 und 7).

Gemäss einem bevorzugten Aspekt des erfindungsgemässen Verfahrens wird das Metallamid $MNR^1R^2$ auf an sich bekannte Art und Weise oder analog bekannter Methoden hergestellt. Wie oben gesagt, ist M vorzugsweise Lithium oder Natrium, insbesondere Lithium. $R^1$ und $R^2$ sind vorzugsweise Äthyl, n-Propyl, n-Butyl oder $R^1$ und $R^2$ bilden zusammen einen carbocyclischen Ring mit einer Methylgruppe in α-Stellung zum Stickstoffatom, also beispielsweise 2-Methylpiperidin oder 2,6-Dimethylpiperidin. Am leichtesten verfügbar ist Diäthylamin, das auch das ökonomischste Amin bildet. Aus diesem Grund ist dieses Amin ganz speziell bevorzugt.

Die Methodik zur Herstellung von Lithium- oder Natriumamiden ausgehend von sekundären Aminen ist bekannt. Als Quelle von aktivem Natrium oder aktivem Lithium können demgemäss beispielsweise benützt werden: Lithiummetall, Natriummetall, Butyllithium, etc.

Speziell bevorzugt ist die Verwendung von Lithiummetall. Die Reaktion zwischen dem Metall und dem Amin kann durch Zugabe einer polycyclischen aromatischen Verbindung, wie beispielsweise Naphthalin oder Biphenyl noch beschleunigt werden, indem in diesem Fall ein löslicher Charge Transfer-[Ladungsübertragung]-Komplex mit dem Metall entsteht (siehe z.B. Organometallic Compounds, Band I, 3. Ausgabe, G.E. Coates u. K. Wade, Methuen, London, 1967, Seite 59).

Für jedes eingesetzte Mol Amin werden zweckmässigerweise 0,005–0,1 Mole Lithium verwendet. Bevorzugt ist eine Menge von 0,01–0,05 Mole Lithium pro Mol Amin und ganz speziell bevorzugt sind 0,02 Mole Lithium pro Mol Amin.

In einer bevorzugten Ausführungsform wird das Metall bzw. die aktive Metallverbindung – beispielsweise Butyllithium – zum Amin gegeben. Obschon ein Lösungsmittel nicht erforderlich ist, kann ein solches reaktionsinertes Lösungsmittel, wie ein Kohlenwasserstoff wie beispielsweise Hexan, Benzol, Toluol etc. verwendet werden.

Myrcen wird hierauf zum Amingemisch gegeben. Die Reaktion ist exothermisch. Die Temperatur kann unter Kontrolle gehalten werden durch die Geschwindigkeit der Zugabe und/oder durch externe Kühlung. Die Reaktion kann in einem Temperaturbereich von ungefähr 0 °C bis ungefähr 100 °C durchgeführt werden. Bevorzugt ist ein Temperaturbereich von ungefähr 25° bis ungefähr 80 °C, wobei Temperaturen von ungefähr 60 °C ganz besonders bevorzugt sind.

Das Verhältnis von Myrcen zu Amin ist nicht kritisch. Bevorzugt ist ein Verhältnis von Myrcen zu Amin, das ungefähr 0,5 bis 2 Mole/Mol beträgt, wobei ein Verhältnis von 0,1 bis 1,2 ganz speziell bevorzugt ist.

Nachdem das Geranylamin entstanden ist, kann dieses entweder hydratisiert oder alkyliert werden, was aus dem unten stehenden Schema III hervorgeht. Aus diesem Schema geht auch hervor, dass die Reihenfolge nicht kritisch ist, da ja in jedem Fall das Endprodukt 7 entsteht.

Schema III

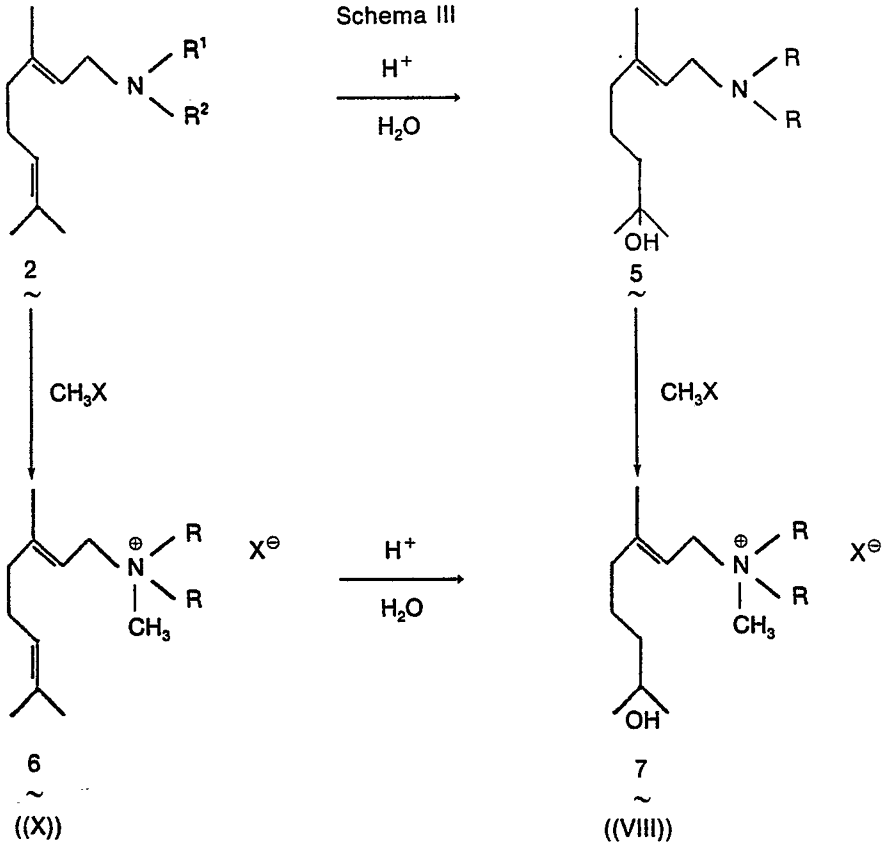


2, 5, 6 ((X)), 7 ((VIII))

Die Hydratisierung wird unter Verwendung von Wasser in Anwesenheit einer Säure bewerkstelligt. Wasser soll vorzugsweise im Überschuss verwendet werden, andere kritische Bedingungen geben sich hier nicht. Es kann jegliche starke Säure, beispielsweise eine solche mit einem pKa-Wert von $<2$ verwendet werden. Bevorzugt sind Schwefelsäure, Salzsäure und Phosphorsäure. Geeignet sind aber auch starke organische Säuren wie Benzolsulfonsäure, Methansulfonsäure oder jegliche starke Säure, die Teil eines löslichen oder kreuzvernetzten Polymeren bildet. Geeignete organische Säuren sind diejenigen der Formel $R^4SO_3H$, worin $R^4$ eine Alkylgruppe mit 1–8 Kohlenstoffatomen darstellt, oder Phenyl, Tolyl oder Teil eines löslichen oder kreuzvernetzten Polymeren bedeutet. Die Stärke der Säure kann zwischen 1–13 molar variieren, ist aber vorzugsweise 5–13 molar. Wenn die Hydratisierung des Amins durchgeführt wird, kann die Menge Säure im Bereiche von 1,1–5 Mole Säure pro Mol Amin betragen, liegt aber vorzugsweise im Bereich von 1,5–2,5 Mol. Für die Hydratisierung des alkylierten Amins (quaternäres Ammoniumsalz) kann die Menge Säure 0,1–2 Mole pro Mol Amin betragen, vorzugsweise liegt sie im Bereich von 0,1–1 Mole pro Mol Amin. Diese Hydratisierung wird vorzugsweise bei Temperaturen, die unter 50 °C aber über 0 °C liegen, durchgeführt.

Die Alkylierung kann nach irgendeiner Standardmethode zur Herstellung von quaternären Salzen durchgeführt werden, also beispielsweise unter Verwendung von Reagenzien wie Dialkylsulfaten ($R_2SO_4$) oder Alkylhalogeniden RX, worin X Chlor, Brom oder Jod darstellt. Im Schema III ist die Alkylgruppe Methyl, aber andere Alkylgruppe wie Methyl bis Octyl, Benzyl etc. sind ebenfalls geeignet. Bevorzugt sind die einfacheren Gruppen wie Methyl oder Äthyl. Ganz speziell bevorzugt ist Methylchlorid oder Dimethylsulfat; diese Agenzien sind billig und immer leicht erhältlich.

Im Falle der Alkylsulfate ist der geeignete Temperaturbereich $-50$ °C bis $+50$ °C, während im Falle der Alkylchloride der Temperaturbereich $+40$ °C bis 120 °C beträgt. Was Benzylchlorid betrifft, ist hier der geeignete Bereich 0–100 °C.

Das Salz wird hierauf ins Hydroxid übergeführt, ein Prozess, der durch jede betreffende der in der Literatur beschriebene Methode bewerkstelligt werden kann, also beispielsweise durch Zugabe eines Äquivalentes oder mehr einer starken Base, wie Natrium- oder Kaliumhydroxid. Das bevorzugte Prozedere besteht in der Zugabe von 1–10 Molen Natriumhydroxid, insbesondere als wässrige Lösung, die 1–30 Gewichtsprozent der Base enthält. Das Hydroxid wird hierauf einer Hofmann-Elimination unterworfen.

Das Salz 7 wird also ins Hydroxid übergeführt und hierauf einem Hofmann'schen Abbau unterworfen. Es existiert eine ganze Reihe von Methoden, um diesen Hofmann'schen Abbau durchzuführen; diese Methoden sind leicht auf die Verbindungen der vorliegenden Erfindung anzuwenden. Eine Methodik geht beispielsweise aus der U.S. Patentschrift 3 932 539 hervor. Der geeignete Temperaturbereich für die thermische Zersetzung beträgt 60–140 °C, wobei 80–120 °C bevorzugt sind.

Das neue Myrcenol/cis-Ocimenol-Gemisch, das auf diese Weise erhalten wird, kann durch einfache Destillation isoliert werden oder aber ohne Destillation mit Acrolein umgesetzt werden. In jedem Falle entsteht das Myrcenol-Acrolein-Addukt und cis-Ocimenol reagiert nicht. Dieses unveränderte cis-Ocimenol kann aus dem Addukt durch Destillation abgetrennt werden, wobei dann ein cis-Ocimenol, das im wesentlichen frei von trans-Ocimenol ist, anfällt.

Das Geranylamin kann also in neue Gemische, die ungefähr 70–95% Myrcenol und ungefähr 50–30% cis-Ocimenol betragen, übergeführt werden.

Die folgenden Beispiele illustrieren die bevorzugten Aspekte der Erfindung, sie sollen nicht limitierend verstanden werden. Sie sollen demgemäss jedes Äquivalent oder naheliegende Variationen der Erfindung die ebenfalls umfassen.

Eine erste Reihe von Beispielen illustriert die bevorzugten Aspekte zur Herstellung der Geranylamine.

Beispiel 1

Herstellung von N,N-Diäthylgeranylamin

84 ml (0,816 Mol) trockenes Diäthylamin wurden in einen 500 ml-Dreihalskolben gegeben, der mit Rückflusskühler, Thermometer, Tropftrichter und Magnetrührer ausgerüstet war. Das Gefäss wurde mit trockenem Stickstoff ausgespült und unter einem Stickstoffkissen gehalten. Hierauf wurden 16,6 ml Lithium (2,2 Mol) in Hexan zugegeben und die Temperatur auf 50° erhöht. Nach 15 Minuten wurden 200 ml 85%iges Myrcen (1 Mol) zugegeben. Es entwickelte sich Reaktionswärme, und es wurde deshalb auf 60° gekühlt. Nach 4 Stunden Reaktion bei 60° zeigte ein Gaschromatogramm, dass das Amin praktisch zu einem einzigen Reaktionsprodukt in hoher Ausbeute umgesetzt worden war. Die Destillation ergab eine Fraktion mit einem Siedepunkt von 108°/7 mmHg, bei welchem Produkt es sich um Diäthylgeranylamin handelte. Die Ausbeute betrug 148,7 g (87% basierend auf dem Amin) das Geranylamin war 96%ig (siehe auch Tabelle I).

Beispiel 2

Das Verfahren des Beispiels 1 wurde wiederholt, wobei 168,5 ml Di-n-butylamin (1 Mol), 250 ml trockenes Myrcen (1.25 Mol) und 2 g 30%iges Lithium in Öl als Lithiumquelle zum Einsatz gelangten. Nach einer Reaktionszeit von 17 Stunden bei 60° resultierte 97%iges N,N-Dibutylgeranylamin vom Siedepunkt 108°/1 mmHg.

Beispiele 3–13

Die Beispiele 3–13 wurden gemäss Verfahren der Beispiele 1 und 2 durchgeführt und sind in der Tabelle I zusammengefasst.

Beispiel 14

Herstellung von N,N-Diäthylgeranylamin

In ein trockenes Reaktionsgefäss wurden 2,3 g einer Lithiumdispersion (30% in Öl) und 10 g Biphenyl gegeben. Das Gefäss wurde mit Stickstoff gespült. Ein Septum (Plastikdeckel) wurde über einen der Hälse gespannt, um die Probenahme mittels Spritze zu erleichtern. Nun wurden 84 g (119 ml) trockenes Diäthylamin zugegeben und das Gemisch 1 Stunde gerührt, wobei eine orangefarbene Lösung resultierte. Es folge nochmals Zugabe von 318 g (450 ml) trockenem Diäthylamin und hierauf von 801,5 g (1 Liter) trockenem 85%igem Myrcen. Die Reaktion war exotherm und die Reaktionstemperatur wurde durch anfängliches Kühlen bei ungefähr 60° gehalten. Nach $6^1/_2$ Stunden war die Konversion über 95%, was mittels Gaschromatographie festgestellt wurde. Nun wurden 500 ml einer gesättigten Natriumchloridlösung und 250 ml Wasser zugegeben. Das Gemisch wurde hierauf in einem Scheidetrichter geschüttelt und die Schichten getrennt. Die organische Schicht wurde nochmals mittels gesättigter Kochsalzlösung (250 ml) gewaschen und abgetrennt. Das Produkt wurde destilliert, wobei 893 g N,N-Diäthylgeranylamin resultierten.

Beispiel 15
Herstellung der 6,7-Dihydro-7-hydroxygeranylamin

In ein 3 l-Reaktionsgefäss wurden 627 g N,N-Diäthylgeranylamin gegeben. Es erfolgte Zugabe von 940 g 62,5%iger Schwefelsäure, und zwar langsam unter heftigem Rühren, um die Temperatur unter 50° zu halten. Das Reaktionsgemisch wurde über Nacht weitergerührt und hierauf mit 1,6 kg 30%iger Sodalösung neutralisiert. Die Schichten wurden getrennt. Die wässrige Phase wurde mit 500 ml Toluol gewaschen. Die organischen Phasen wurden kombiniert, mit 500 ml einer 50%igen gesättigten Salzlösung gewaschen, über Magnesiumsulfat getrocknet, filtriert und destilliert, wobei 572,5 g N,N-Diäthyl-(6,7-dihydro-7-hydroxygeranyl)amin vom Siedepunkt 105–107°/2 mmHg anfielen. Die Ausbeute betrug 84%.

Beispiel 16
Herstellung von Myrcenol

Zu 227 g N,N-Diäthyl-(6,7-dihydro-7-hydroxygeranyl)amin in 1 Liter Toluol wurden langsam innert 1–2 Stunden 126 g Dimethylsulfat gegeben. Das Reaktionsgemisch wurde $^1/_2$ Stunde gerührt. Hierauf wurden die zwei Phasen getrennt. Die obere Phase wurde dreimal mit je 200 ml Wasser extrahiert und die wässrigen Extrakte zur unteren Phase gegeben. Das wässrige Gemisch wurde langsam zu 3 Liter 30%igem Natriumhydroxyd, welches bei Rückflusstemperatur gehalten wurde, zugetropft und das resultierende Produkt mittels Wasserdampfdestillation gesammelt. Das Wasserdampfdestillat wurde hintereinander mit 100 ml 50%iger Kochsalzlösung, mit 100 ml 10%iger Essigsäure, mit 100 ml Wasser, mit 100 ml 10%igem Natriumbicarbonat und nochmals mit 100 ml Wasser gewaschen. Das nun resultierende Produkt bestand aus ungefähr 76,2% Myrcenol, 23,8% cis-Ocimenol, und 2% oder sogar weniger trans-Ocimenol; die Ausbeute betrug 78,1%.

Beispiel 17
Myrcenol

60 g Methanol und 113,5 g N,N-Diäthyl-(6,7-dihydro-7-hydroxygeranyl)amin wurden in einem 300 ml-Autoklaven gegeben. Dazu kamen 27 g Methylchlorid; nun wurde auf 80° erhitzt und zwar 3–6 Stunden, nach welcher Zeit die Reaktion beendigt war. Der Reaktor wurde gekühlt, ventiliert und das zurückbleibende Methylchlorid unter schwachem Vakuum entfernt. Das rohe quaternäre Ammoniumsalz wurde in eine für eine Dampfdestillation geeignete Apparatur gegeben und 70 g 30%iges Natriumhydroxid zugegeben. Das Gemisch wurde dampfdestilliert, bei einer Gefässtemperatur von 120–150°. Als kein organisches Material mehr überging, wurde das Destillat mit 140 g Hexan extrahiert. Die Hexanlösung wurde mit 100 ml einer gesättigten wässrigen Natriumchloridlösung gewaschen. Eine Vakuumdestillation lieferte 66,1 g eines Gemisches von 88,5% Myrcenol, 9,3% cis-Ocimenol und 2,2% trans-Ocimenol.

Beispiel 18
Herstellung von 3- und 4-(4-Methyl-4-hydroxyamyl)$\Delta^3$-cyclohexen-carboxaldehyd

In ein Druckgefäss wurden 77 g des Myrcenolgemisches des Beispiels 17, 56 g Acrolein und 1 g Hydrochinon gegeben. Der Reaktor wurde mit Stickstoff gespült, verschlossen und 4 Stunden auf 150° erhitzt. Eine Destillation des resultierenden Gemisches ergab 3- und 4-(4-Methyl-4-hydroxyamyl)$\Delta^3$-cyclohexen-carboxaldehyd zusammen mit nicht umgesetztem cis-Ocimenol.

Beispiel 19
Reinigung des cis-Ocimenols

Das cis-Ocimenol des Beispiels 18 [das geringe Mengen Myrcenol oder trans-Ocimenol enthalten kann] wurde durch Reaktion mit dem dienophilen Maleinsäureanhydrid weitergereinigt. Maleinsäureanhydrid wurde deshalb zugegeben, weil dies allfälliges zurückbleibendes Myrcenol oder trans-Ocimenol bindet und deshalb die Destillation des reinen cis-Ocimenols aus dem Gemisch erlaubt]. Das Reaktionsgemisch wurde mit wässriger Natriumhydroxidlösung gewaschen und destilliert. Auf diese Weise resultierte cis-Ocimenol in einer Reinheit von mindestens 98%, was durch gaschromatographische Analyse etabliert wurde.

**Patentansprüche**

1. Verfahren zur Herstellung von Riechstoffgemischen, dadurch gekennzeichnet, dass man Myrcen mit einem sekundären Amin der Formel

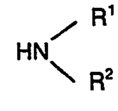

VII

worin $R^1$ und $R^2$ gleich oder verschieden sind und Äthyl oder einen höheren aliphatischen Rest darstellen bzw. zusammen eine cycloaliphatische

Gruppe mit einem 1–8 Kohlenstoffatome enthaltenden Substituenten an einem der an den Stickstoff gebundenen Kohlenstoffatome bedeuten, in Anwesenheit des entsprechenden Alkalimetallamids umsetzt,

b) das erhaltene Geranylamin der Formel

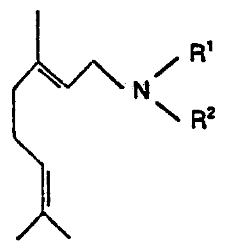
V

durch Hydratisierung und Alkylierung in beliebiger Reihenfolge, gefolgt von Behandlung mit einer starken Base, in das entsprechende E-3,7-Dimethyl-7-hydroxy-2-octen-1-yl quaternäre Ammoniumhydroxid der Formel

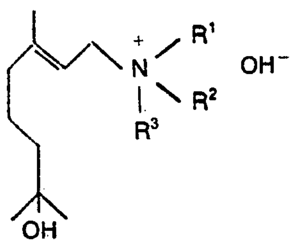
VIII′

worin $R^3$ einen Alkylrest mit 1–8 Kohlenstoffatomen darstellt oder für einen unsubstituierten oder substituierten Benzylrest steht und $R^1$ und $R^2$ obige Bedeutung besitzt, überführt,

c) die erhaltene Verbindung der Formel VIII′ den Bedingungen des Hofmann'schen Abbaus unterwirft, um zu einem Myrcenol/cis-Ocimenol-Gemisch, das im wesentlichen frei von trans-Ocimenol ist, zu gelangen, und dieses Myrcenol/cis-Ocimenol-Gemisch mit Acrolein unter den Bedingungen der Diels-Alder-Reaktion zu einem Isomerengemisch von 3- und 4-(4-Methyl-4-hydroxy-amyl)-$\Delta^3$-cyclohexencarboxaldehyd umsetzt, und aus dem Gemisch nicht umgesetztes cis-Ocimenol abtrennt.

2. Verfahren nach Anspruch 1, dadurch gekennzeichnet, dass $R^1$ und $R^2$ Äthyl, n-Propyl oder n-Butyl oder zusammen mit dem Stickstoffatom, an das sie gebunden sind, 2-Methylpiperidyl darstellen, und Lithium oder Natrium als Alkalimetall verwendet werden.

3. Verfahren nach Anspruch 1 oder 2, dadurch gekennzeichnet, dass das Geranylamin

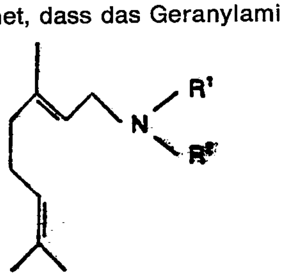
V

durch Reaktion mit einer 1–13 molaren wässrigen Lösung von Schwefelsäure, Phosphorsäure oder Salzsäure oder einer 1–13 molaren Lösung einer organischen Sulfonsäure der Formel $R^4SO_3H$, worin $R^4$ einen $C_{1-8}$-Alkylrest, Phenyl, Tolyl oder Teil eines löslichen oder kreuzvernetzten Polymeren darstellt, in das 7-Hydroxygeranylamin der Formel

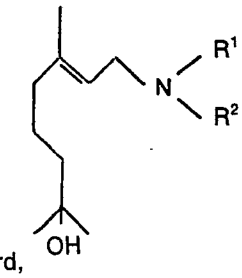
IX

übergeführt wird,

b) und dieses 7-Hydroxygeranylamin durch Reaktion mit Dialkylsulfat, Alkylchlorid, Alkylbromid, Alkyljodid, Benzylchlorid, Benzylbromid oder Benzyljodid in eine Verbindung der Formel

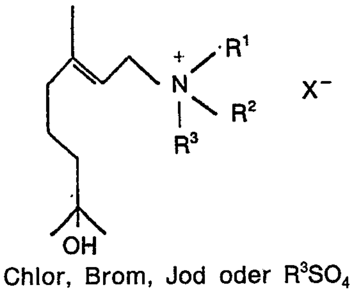
VIII

worin X Chlor, Brom, Jod oder $R^3SO_4$ darstellt, übergeführt wird.

4. Verfahren nach Anspruch 1 oder 2, dadurch gekennzeichnet, dass das Geranylamin

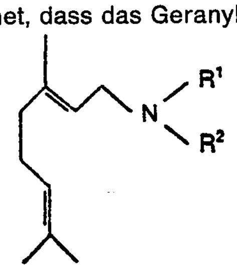
V

durch Reaktion mit Dialkylsulfat, Alkylchlorid, Alkylbromid, Alkyljodid, Benzylchlorid, Benzylbromid oder Benzyljodid in das entsprechende E-3,7-Dimethyl-2,6-octadien-1-yl quaternäre Ammoniumsalze der Formel

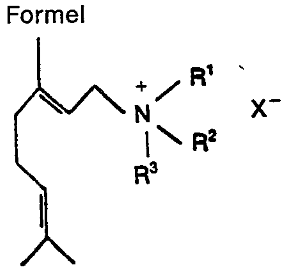
X

worin X Chlor, Brom, Jod oder $R^3SO_4$ darstellt, übergeführt wird,

b) und das E-3,7-Dimethyl-2,6-octadien-1-yl quaternäre Ammoniumsalz durch Reaktion mit einer 1–13 molaren wässrigen Lösung von Schwefelsäure, Phosphorsäure, Salzsäure oder einer organischen Sulfonsäure der Formel $R^4SO_3H$, worin $R^4$ einen $C_{1-8}$-Alkylrest Phenyl, Tolyl oder Teil eines löslichen oder kreuzvernetzten Polymers darstellt, in die Verbindung der Formel VIII gemäss Anspruch 3 übergeführt wird.

5. Verfahren nach Anspruch 3, dadurch gekennzeichnet, dass

a) Myrcen mit Diäthylamin, Dipropylamin oder α-Methylpiperidin umgesetzt wird,

b) das erhaltene Geranylamin mit einer 1–13 molarer Schwefelsäure, Phosphorsäure oder Salzsäure bei Temperaturen zwischen 0° und 50 °C umgesetzt wird, und

c) das erhaltene 7-Hydroxygeranylamin mit

i) einem $C_{1-8}$-Alkylchlorid, -bromid oder -jodid bei Temperaturen zwischen 40 und 120 °C, oder mit

ii) Benzylchlorid, -bromid oder -jodid bei Temperaturen zwischen 0° und 100 °C, oder mit

iii) Dimethylsulfat oder Diäthylsulfat bei Temperaturen zwischen −50 und +50 °C umgesetzt wird.

6. Verfahren nach Anspruch 4, dadurch gekennzeichnet, dass

a) Myrcen mit Diäthylamin, Dipropylamin oder 2-Methylpiperidin umgesetzt wird,

b) das erhaltene Geranylamin mit

i) einem $C_{1-8}$-Alkylchlorid, -bromid oder -jodid bei Temperaturen zwischen 40 und 120 °C, oder mit

ii) Benzylchlorid, -bromid oder -jodid bei Temperaturen zwischen 0 und 100 °C, oder mit

iii) Dimethylsulfat oder Diäthylsulfat bei Temperaturen zwischen −50 und +50 °C umgesetzt wird, und

c) das erhaltene E-3,7-Dimethyl-2,6-octadienyl quaternäre Ammoniumsalz mit 1–13 molarer Schwefelsäure, Phosphorsäure oder Salzsäure bei Temperaturen zwischen 0 und 50 °C umgesetzt wird.

7. Verfahren nach Anspruch 5 oder 6, dadurch gekennzeichnet, dass das quaternäre Ammoniumsalz der Formel

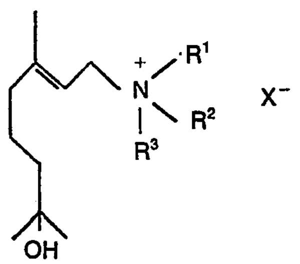 VIII

bei Temperaturen zwischen 60 und 140 °C zu einem bis 10 Äquivalenten Natrium- oder Kaliumhydroxid gegeben wird.

8. Verfahren nach Anspruch 5, dadurch gekennzeichnet, dass

a) bei der Herstellung von Lithium-diäthylamid 0,01 bis 0,05 Mol Lithium pro Mol Amin verwendet werden,

b) 0,8 bis 1,2 Mol Äquivalente Myrcen bei einer Temperatur von 25–80 °C zum Lithium-diäthylamid gegeben werden,

c) das erhaltene N,N-Diäthylgeranylamin bei einer Temperatur von 0–50 °C mit 1,1–5 Mol 1,0–13 molarer Schwefelsäure pro Mol Amin umgesetzt wird,

d) das erhaltene 7-Hydroxygeranylamin bei einer Temperatur von 40–120 °C mit Methylchlorid alkyliert wird,

e) das 7-Hydroxygeranyl-diäthylmethylammoniumchlorid mit 1–10 Äquivalenten Natriumhydroxid umgesetzt wird, und

f) das E-3,7-Dimethyl-7-hydroxy, 2-octen-1-yl-diäthylmethylammoniumhydroxid auf eine Temperatur von 60–140 °C erhitzt wird.

9. Verfahren nach Anspruch 6, dadurch gekennzeichnet, dass

a) bei der Herstellung von Lithiumdiäthylamid 0,01 bis 0,05 Mol Lithium pro Mol Amin zum Einsatz gelangen,

b) 0,8 bis 1,2 Mol-Äquivalente von Myrcen bei einer Temperatur zwischen 25–80 °C zum Lithiumdiäthylamin gegeben werden,

c) das erhaltene N,N-Diäthylgeranylamin mittels Methylchlorid in einem Temperaturbereich von 40–120 °C alkyliert wird,

d) das E-3,7-Dimethyl-2,6-octadienyl quaternäre Ammoniumsalz mit 0,1 bis 2 Molen einer 1,0 bis 13 molaren Schwefelsäure pro Mol Amin bei einer Temperatur von 0–50 °C umgesetzt wird,

e) das 7-Hydroxygeranyl-diäthylmethylammoniumchlorid mit einem bis 10 Äquivalenten Natriumhydroxid umgesetzt wird, und

f) das erhaltene E-3,7-Dimethyl-7-hydroxy-2-octen-1-yl-diäthylmethylammoniumhydroxid auf eine Temperatur von 60–140 °C erhitzt wird.

10. Verfahren nach Anspruch 8 oder 9, dadurch gekennzeichnet, dass der Hofmann'sche Abbau zwischen 80 und 120 °C durchgeführt wird.

## Claims

1. A process for the manufacture of odorant mixtures, characterized by reacting myrcene with a secondary amine of the formula

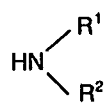 VII

wherein $R^1$ and $R^2$ are the same or different and represent ethyl or a higher aliphatic residue or $R^1$ and $R^2$ together signify a cycloaliphatic group with a substituent containing 1–8 carbon atoms on one of the carbon atoms bonded to the nitrogen,
in the presence of the corresponding alkali metal amide,

b) converting the resulting geranylamine of the formula

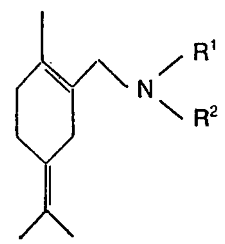 V

by hydration and alkylation in optional sequence, followed by treatment with a strong base, into the corresponding E-3,7-dimethyl-7-hydroxy-2-octen-1-yl quaternary ammonium hydroxide of the formula

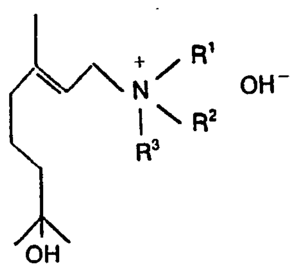

VIII′

wherein $R^3$ represents an alkyl residue with 1–8 carbon atoms or stands for an unsubstituted or substituted benzyl residue and $R^1$ and $R^2$ have the above significance,

c) subjecting the resulting compound of formula VIII′ to the conditions of a Hofmann elimination in order to provide a myrcenol/cis-ocimenol mixture, which is substantially free from trans-ocimenol, and reacting this myrcenol/cis-ocimenol mixture with acrolein under the conditions of a Diels-Alder reaction to give an isomer mixture of 3- and 4-(4-methyl-4-hydroxyamyl)-$\Delta^3$-cyclohexenecarboxaldehyde and separating unreacted cis-ocimenol from the mixture.

2. A process according to claim 1, characterized in that $R^1$ and $R^2$ represent ethyl, n-propyl or n-butyl or $R^1$ and $R^2$ together with the nitrogen atom to which they are attached represent 2-methylpiperidyl and lithium or sodium is used as the alkali metal.

3. A process according to claim 1 or 2, characterized in that the geranylamine

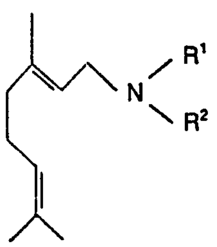

V

is converted by reaction with a 1–13 molar aqueous solution of sulphuric acid, phosphoric acid or hydrochloric acid or a 1–13 molar solution of an organic sulphonic acid of the formula $R^4SO_3H$, wherein $R^4$ represents a $C_{1-8}$-alkyl residue, phenyl, tolyl or part of a soluble or cross-linked polymer, into the 7-hydroxygeranylamine of the formula

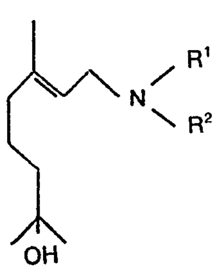

IX

b) and this 7-hydroxygeranylamine is converted by reaction with dialkyl sulphate, alkyl chloride, alkyl bromide, alkyl iodide, benzyl chloride, benzyl bromide or benzyl iodide into a compound of the formula

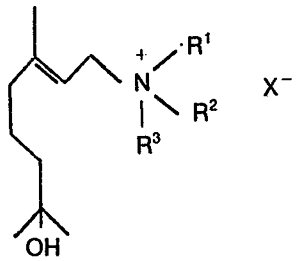

VIII

wherein X represents chlorine, bromine, iodine or $R^3SO_4$.

4. A process according to claim 1 or 2, characterized in that the geranylamine

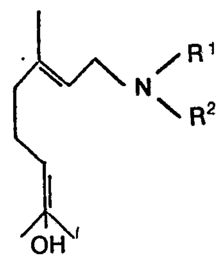

V

is converted by reaction with dialkyl sulphate, alkyl chloride, alkyl bromide, alkyl iodide, benzyl chloride, benzyl bromide or benzyl iodide into the corresponding E-3,7-dimethyl-2,6-octadien-1-yl quaternary ammonium salt of the formula

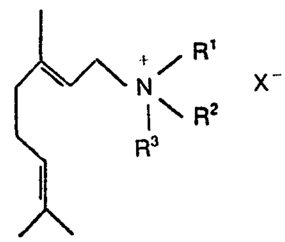

X

wherein X represents chlorine, bromine, iodine or $R^3SO_4$,

b) and the E-3,7-dimethyl-2,6-octadien-1-yl quaternary ammonium salt is converted by reaction with a 1–13 molar aqueous solution of sulphuric acid, phosphoric acid, hydrochloric acid or an organic sulphonic acid of the formula $R^4SO_3H$, wherein $R^4$ represents a $C_{1-8}$-alkyl residue, phenyl, tolyl or part of a soluble or cross-linked polymer, into the compound of formula VIII in accordance with claim 3.

5. A process according to claim 3, characterized in that

a) myrcene is reacted with diethylamine, dipropylamine or α-methylpiperidine,

b) the geranylamine obtained is reacted with a 1–13 molar sulphuric acid, phosphoric acid or hydrochloric acid at temperatures between 0° and 50 °C, and

c) the 7-hydroxygeranylamine obtained is reacted with

i) a $C_{1-8}$-alkyl chloride, bromide or iodide at temperatures between 40 and 120 °C, or with

ii) benzyl chloride, bromide or iodide at temperatures between 0° and 100 °C, or with

iii) dimethyl sulphate or diethyl sulphate at temperatures between −50 and +50 °C.

6. A process according to claim 4, characterized in that

a) myrcene is reacted with diethylamine, dipropylamine or 2-methylpiperidine,

b) the geranylamine obtained is reacted with

i) a $C_{1-8}$-alkyl chloride, bromide or iodide at temperatures between 40 and 120 °C, or with

ii) benzyl chloride, bromide or iodide at temperatures between 0 and 100 °C, or with

iii) dimethyl sulphate or diethyl sulphate at temperatures between −50 and +50 °C, and

c) the E-3,7-dimethyl-2,6-octadienyl quaternary ammonium salt obtained is reacted with 1–13 molar sulphuric acid, phosphoric acid or hydrochloric acid at temperatures between 0 and 50 °C.

7. A process according to claim 5 or 6, characterized in that the quaternary ammonium salt of the formula

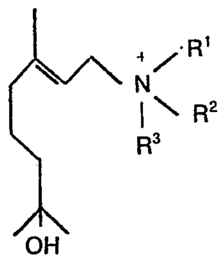 $X^-$ VIII

is added to one to 10 equivalents of sodium or potassium hydroxide at temperatures between 60 and 140 °C.

8. A process according to claim 5, characterized in that

a) 0.01 to 0.05 mole of lithium are used per mole of amine in the preparation of lithium diethylamide,

b) 0.8 to 1.2 mole equivalents of myrcene are added to the lithium diethylamide at a temperature of 25–80 °C,

c) the N,N-diethylgeranylamine obtained is reacted with 1.1–5 mole of 1.0–13 molar sulphuric acid per mole of amine at a temperature of 0–50 °C,

d) the 7-hydroxygeranylamine obtained is alkylated with methyl chloride at a temperature of 40–120 °C,

e) the 7-hydroxygeranyl diethylmethylammonium chloride is reacted with 1–10 equivalents of sodium hydroxide, and

f) the E-3,7-dimethyl-7-hydroxy, 2-octen-1-yl diethylmethylammonium hydroxide is heated to a temperature of 60–140 °C.

9. A process according to claim 6, characterized in that

a) 0.01 to 0.05 mole of lithium are used per mole of amine in the preparation of lithium diethylamide,

b) 0.8 to 1.2 mole equivalents of myrcene are added to the lithium diethylamine at a temperature between 25–80 °C,

c) the N,N-diethylgeranylamine obtained is alkylated by means of methyl chloride in a temperature range of 40–120 °C,

d) the E-3,7-dimethyl-2,6-octadienyl quaternary ammonium salt is reacted with 0.1 to 2 moles of a 1.0 to 13 molar sulphuric acid per mole of amine at a temperature of 0–50 °C,

e) the 7-hydroxygeranyl diethylmethylammonium chloride is reacted with one to 10 equivalents of sodium hydroxide, and

f) the E-3,7-dimethyl-7-hydroxy-2-octen-1-yl diethylmethylammonium hydroxide obtained is heated to a temperature of 60–140 °C.

10. A process according to claim 8 or 9, characterized in that the Hofmann elimination is carried out between 80 and 120 °C.

## Revendications

1. Procédé de préparation de mélanges de matières odorantes caractérisé en ce que l'on fait réagir le myrcène avec une amine secondaire de formule

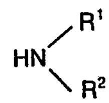 VII

dans laquelle $R^1$ et $R^2$, ayant des significations identiques ou différentes, représentent chacun un groupe éthyle ou un radical aliphatique supérieur, ou forment ensemble un groupe cycloaliphatique portant un substituant contenant 1 à 8 atomes de carbone sur l'un des atomes de carbone reliés à l'azote,

en présence de l'amidure de métal alcalin correspondant,

b) on convertit la géranylamine ainsi obtenue, répondant à la formule

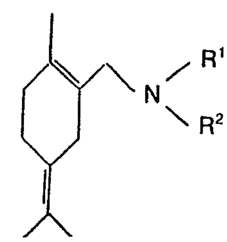 V

par hydratation et alkylation dans un ordre quelconque, suivies du traitement par une base forte, en l'hydroxyde de E-3,7-diméthyl-7-hydroxy-2-octène-1-yl-ammonium quaternaire correspondant à la formule

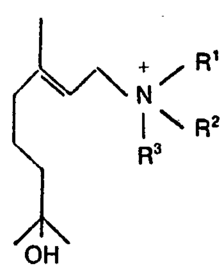 $OH^-$ VIII'

dans laquelle $R^3$ représente un groupe alkyle en C1–C8 ou bien un groupe benzyle non substitué ou substitué et $R^1$ et $R^2$ ont les significations indiquées ci-dessus,

c) on soumet le composé obtenu, répondant à la formule VIII', aux conditions de la dégradation de Hofmann, ce qui donne un mélange myrcénol/cis-

ociménol essentiellement exempt de trans-ociménol, et on fait réagir ce mélange myrcénol/cis-ociménol avec l'acroléine dans les conditions de la réaction de Diels-Alder, ce qui donne un mélange de 3- et 4-(4-méthyl-4-hydroxyamil)-$\Delta^3$-cyclohexène-carboxaldéhydes isomères, d'où l'on sépare le cis-ociménol non converti.

2. Procédé selon la revendication 1, caractérisé en ce que $R^1$ et $R^2$ représentent des groupes éthyle, n-propyle ou n-butyle ou forment ensemble et avec l'atome d'azote auxquels ils sont reliés un groupe 2-méthyl-pipéridyle, et en ce que l'on utilise en tant que métal alcalin le lithium ou le sodium.

3. Procédé selon la revendication 1 ou 2, caractérisé en ce que l'on convertit la géranylamine

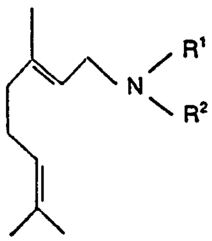

V

par réaction avec une solution aqueuse à une concentration 1 à 13 M d'acide sulfurique, d'acide phosphorique ou d'acide chlorhydrique ou avec une solution à une concentration 1 à 13 M d'un acide sulfonique organique de formule $R^4SO_3H$ dans laquelle $R^4$ représente un groupe alkyle en C1–C8, phényle, tolyle ou la partie correspondante d'un polymère soluble ou réticulé, en la 7-hydroxygéranylamine de formule

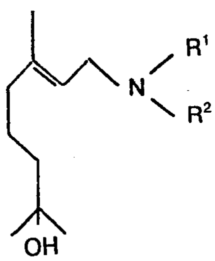

IX

b) et on convertit cette 7-hydroxygéranylamine, par réaction avec un sulfate de dialkyle, un chlorure d'alkyle, un bromure d'alkyle, un iodure d'alkyle, le chlorure de benzyle, le bromure de benzyle ou l'iodure de benzyle, en un composé de formule

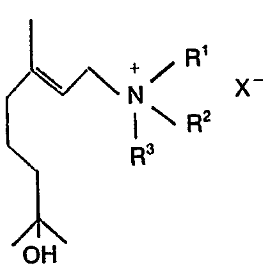

VIII

dans laquelle X représente le chlore, le brome, l'iode ou un groupe $R^3SO_4$.

4. Procédé selon la revendication 1 ou 2, caractérisé en ce que l'on convertit la géranylamine

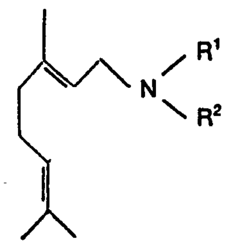

V

par réaction avec un sulfate de dialkyle, un chlorure d'alkyle, un bromure d'alkyle, un iodure d'alkyle, le chlorure de benzyle, le bromure de benzyle ou l'iodure de benzyle en le sel de E-3,7-diméthyl-2,6-octadiène-1-yl-ammonium quaternaire correspondant de formule

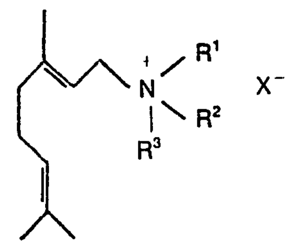

X

dans laquelle X représente le chlore, le brome, l'iode ou le groupe $R^3SO_4$,

b) et on convertit le sel d'E-3,7-diméthyl-2,6-octadiène-1-yl-ammonium quaternaire, par réaction avec une solution aqueuse à une concentration 1 à 13 M d'acide sulfurique, d'acide phosphorique, d'acide chlorhydrique ou d'un acide sulfonique organique de formule $R^4SO_3H$ dans laquelle $R^4$ représente un groupe alkyle en C1–C8, phényle, tolyle, ou la partie correspondante d'un polymère soluble ou réticulé, en le composé de formule VIII selon la revendication 3.

5. Procédé selon la revendication 3, caractérisé en ce que

a) on fait réagir le myrcène avec la diéthylamine, la dipropylamine ou l'alpha-méthylpipéridine,

b) on fait réagir la géranylamine obtenue avec une solution 1 à 13 M d'acide sulfurique, d'acide phosphorique ou d'acide chlorhydrique à des températures de 0 à 50 °C et

c) on fait réagir la 7-hydroxygéranylamine ainsi obtenue avec

i) un chlorure, bromure ou iodure d'alkyle en C1–C8 à des températures de 40 à 120 °C, ou bien

ii) le chlorure, le bromure ou l'iodure de benzyle à des températures de 0 à 100 °C; ou bien

iii) le sulfate de diméthyle ou le sulfate de diéthyle à des températures de −50 à +50 °C.

6. Procédé selon la revendication 4, caractérisé en ce que

a) on fait réagir le myrcène avec la diéthylamine, la dipropylamine ou la 2-méthylpipéridine,

b) on fait réagir la géranylamine ainsi obtenue avec

i) un chlorure, bromure ou iodure d'alkyle en C1–C8 à des températures de 40 à 120 °C, ou bien

ii) le chlorure, le bromure ou l'iodure de benzyle à des températures de 0 à 100 °C, ou bien

iii) le sulfate de diméthyle ou le sulfate de diéthyle à des températures de −50 à +50 °C, et

c) on fait réagir le sel d'E-3,7-diméthyl-2,6-octadiényl-ammonium quaternaire ainsi obtenu avec de l'acide sulfurique, de l'acide phosphorique ou de l'acide chlorhydrique à une concentration 1 à 13 M à des températures de 0 à 50 °C.

7. Procédé selon la revendication 5 ou 6, caractérisé en ce que l'on ajoute le sel d'ammonium quaternaire de formule

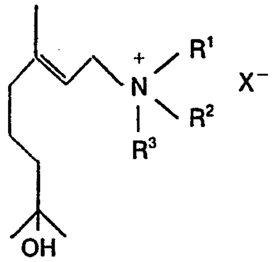

VIII

à des températures de 60 à 140 °C, à de l'hydroxyde de sodium ou de potassium en quantité de 1 à 10 équivalents.

8. Procédé selon la revendication 5, caractérisé en ce que

a) à la préparation du diéthylamidure de lithium, on utilise de 0,01 à 0,05 mole de lithium par mole d'amine,

b) on ajoute au diéthylamidure de lithium, à une température de 25 à 80 °C, de 0,8 à 1,2 équivalents molaires de myrcène,

c) on fait réagir la N,N-diéthylgéranylamine ainsi obtenue à une température de 0 à 50 °C avec 1,1 à 5 moles d'acide sulfurique à une concentration 1,0 à 13 M par mole d'amine,

d) on alkyle la 7-hydroxygéranylamine ainsi obtenue par le chlorure de méthyle à une température de 40 à 120 °C,

e) on fait réagir le chlorure de 7-hydroxygéranyl-diéthylméthylammonium avec 1 à 10 équivalents d'hydroxyde de sodium, et

f) on chauffe l'hydroxyde d'E-3,7-diméthyl-7-hydroxy-2-octène-1-yl-diéthylméthylammonium à une température de 60 à 140 °C.

9. Procédé selon la revendication 6, caractérisé en ce que

a) à la préparation du diéthylamidure de lithium, on utilise de 0,01 à 0,05 mole de lithium par mole d'amine,

b) on ajoute au diéthylamidure de lithium, à une température de 25 à 80 °C, de 0,8 à 1,2 équivalents molaires de myrcène,

c) on alkyle la N,N-diéthylgéranylamine ainsi obtenue dans un intervalle de température de 40 à 120 °C par le chlorure de méthyle,

d) on fait réagir le sel d'E-3,7-diméthyl-2,6-octadiényl-ammonium quaternaire avec 0,1 à 2 moles d'un acide sulfurique, 1,0 à 13 M par mole d'amine à une température de 0 à 50 °C,

e) on fait réagir le chlorure de 7-hydroxygéranyl-diéthylméthylammonium avec 1 à 10 équivalents d'hydroxyde de sodium, et

f) on chauffe l'hydroxyde d'E-3,7-diméthyl-7-hydroxy-2-octène-1-yl-diéthylméthylammonium ainsi obtenu à une température de 60 à 140 °C.

10. Procédé selon la revendication 8 ou 9, caractérisé en ce que la dégradation de Hofmann est effectuée entre 80 et 120 °C.